(19) Europäisches Patentamt
European Patent Office
Office européen des brevets

(11) Numéro de publication : **0 065 468**
**B1**

(12) # FASCICULE DE BREVET EUROPÉEN

(45) Date de publication du fascicule du brevet : **24.04.85**

(21) Numéro de dépôt : **82400914.6**

(22) Date de dépôt : **17.05.82**

(51) Int. Cl.⁴ : **C 07 B 33/00**, C 07 B 35/00, C 07 J 1/00, C 07 J 5/00, C 07 J 7/00, C 07 J 9/00

(54) **Nouveaux agents de déshydrogénation à base de dérivés organiques du sélénium et leur utilisation à la déshydrogénation en 1,4 de stéroïdes.**

(30) Priorité : **19.05.81 FR 8109917**

(43) Date de publication de la demande : **24.11.82 Bulletin 82/47**

(45) Mention de la délivrance du brevet : **24.04.85 Bulletin 85/17**

(84) Etats contractants désignés : **AT BE CH DE FR GB IT LI LU NL SE**

(56) Documents cités :
**US-A- 4 220 588**
**JOURNAL OF THE CHEMICAL SOCIETY, PERKIN TRANSACTIONS I, 1980, no.10, Londres (GB) D.H.R. BARTON et al.: "Dehydrogenation of steroidal- and triterpenoid ketones using benzene-seleninic anhydride", pages 2209-2272**
**JOURNAL OF THE CHEMICAL SOCIETY CHEMICAL COMMUNICATIONS, no.3, 1er février 1978, Londres (GB) D.H.R. BARTON et al.: "Dehydrogenation of steroidal ketones using benzeneselenic anhydride", pages 130-131**
**HELVETICA CHIMICA ACTA, vol.39, no.3, 2 mai 1956, Bale (CH) CH. MEYSTRE et al.: "Gewinnung von 1,4-bisdehydro-3-oxo-steroiden", pages 734-742**
**JOURNAL OF THE CHEMICAL SOCIETY, CHEMICAL COMMUNICATIONS, no.20, octobre 1981, Londres (GB) D.H.R. BARTON et al.: "Oxygen atom transfer from iodylbenzene to Diphenyl Diselenide - A convenient method for dehydrogenation of steroidal 3-ketones", pages 1044-1045**

(73) Titulaire : **ROUSSEL-UCLAF**
**35, boulevard des Invalides**
**F-75007 Paris (FR)**

(72) Inventeur : **Barton, Derek Harold Richard**
**Bât. A/B/C/, route du Château Fort**
**F-91190 Gif Sur Yvette (FR)**
Inventeur : **Motherwell, William Branks Bât. 3, Appt.41**
**Résidence des Quinconces Route du Château Fort**
**F-91190 Gif Sur Yvette (FR)**

(74) Mandataire : **Tonnellier, Marie-José et al**
**ROUSSEL-UCLAF 111, route de Noisy Boîte Postale no.9**
**F-93230 Romainville (FR)**

EP 0 065 468 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne de nouveaux agents de déshydrogénation à base de dérivés organiques du sélénium et leur utilisation à la déshydrogénation en 1,4 de stéroïdes.

L'invention a pour objet, à titre d'agents de déshydrogénation, certains dérivés organiques du sélénium, lorsqu'ils sont utilisés en présence d'un agent d'oxydation, choisi dans le groupe constitué par les dérivés oxydés des composés iodoaromatiques éventuellement substitués.

Il est connu d'utiliser les dérivés organiques du sélénium comme agents de déshydrogénation. C'est ainsi qu'il est possible en particulier de déshydrogéner en 1,4 des stéroïdes portant une fonction cétone en 3 (selon le procédé indiqué par DHR Barton et Coll dans J. Chem. Soc. Perkin I 1980 p 2209 et suivantes) en utilisant l'anhydride benzène séléninique ; toutefois, cette méthode présente deux inconvénients importants :

a) C'est une méthode coûteuse, du fait du prix élevé de l'anhydride benzène séléninique,

b) C'est une méthode qui donne lieu à des réactions secondaires dues à la formation d'acide sélénique ou d'autres dérivés provenant de la réduction de l'anhydride benzène séléninique.

Ces deux inconvénients rendent la méthode difficile à utiliser sur le plan industriel.

On vient de découvrir qu'il est possible d'utiliser industriellement des dérivés organiques du sélénium comme agent de déshydrogénation.

L'invention a notamment pour objet à titre d'agents de déshydrogénation, le diphényl disélénure et l'anhydride benzène séléninique lorsqu'ils sont utilisés en quantité catalytique, en présence d'un agent d'oxydation choisi dans le groupe constitué par l'iodoxybenzène, l'acide méta-iodoxybenzoïque et les composés de formule

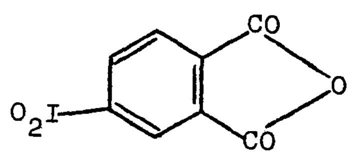 et 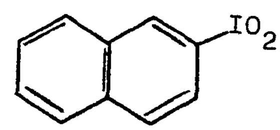

L'invention réside notamment dans le fait que l'on utilise un agent d'oxydation pour oxyder la forme réduite du dérivé organique du sélénium utilisé. Il est alors possible d'utiliser des quantités catalytiques du dérivé de sélénium, ce qui rend le procédé économiquement rentable.

Le dérivé organique du sélénium, après avoir servi à la déshydrogénation, est aussitôt oxydé à nouveau : il n'y a donc pas de formation de réactions secondaires dues à la formation d'acide sélénique ou d'autres dérivés provenant de la réduction des dérivés du sélénium utilisé.

On peut donc schématiser ainsi la réaction :

Forme réduite du dérivé du sélénium en quantité catalytique

↓ + agent d'oxydation

Forme oxydée du dérivé du sélénium

↓ déshydrogénation

L'invention a plus particulièrement pour objet, à titre d'agent de déshydrogénation, le diphényldisélénure lorsqu'il est utilisé en quantité catalytique, en présence d'un agent d'oxydation, tel que défini ci-dessus.

Le mécanisme d'action est le suivant :

Diphényl disélénure + agent d'oxydation → anhydride benzène séléninique → (déshydrogénation) diphényl disélénure

L'invention consiste donc à former « in situ » l'anhydride benzène séléninique.

Il est très intéressant d'un point de vue économique d'utiliser le diphényl disélénure, mais l'on peut également utiliser l'anhydride benzène séléninique lui-même.

L'invention a donc également pour objet l'anhydride benzène séléninique utilisé en quantité catalytique en présence d'un agent d'oxydation tel que défini ci-dessus.

L'invention a plus particulièrement pour objet, à titre d'agent de déshydrogénation, le diphényl disélénure utilisé en quantité catalytique en présence d'acide métaiodoxybenzoïque.

Il est particulièrement intéressant d'utiliser l'acide méta-iodoxybenzoïque comme agent d'oxydation, car il est possible de récupérer l'acide méta-iodoxybenzoïque et le diphényl disélénure non utilisé dans la réaction, sans chromatographie comme il est indiqué plus loin dans la partie expérimentale.

Les agents de déshydrogénation de l'invention sont principalement adaptés à la déshydrogénation en 1,4 des stéroïdes ou des produits chimiques apparentés.

L'invention a ainsi pour objet l'utilisation des agents de déshydrogénation tels que définis précédemment, caractérisée en ce que l'on soumet un composé de formule I

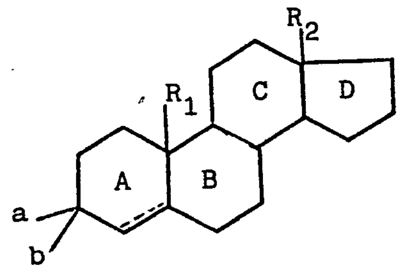

(I)

dans laquelle $R_1$ représente un atome d'hydrogène, un radical alkyle renfermant de 1 à 4 atomes de carbone éventuellement substitué par une fonction oxygénée ou azotée ou un atome d'halogène, ou $R_1$ représente un radical alkényle ou alkynyle renfermant de 2 à 4 atomes de carbone, $R_2$ représente un radical alkyle renfermant de 1 à 4 atomes de carbone, le radical

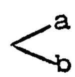

représente soit le groupement

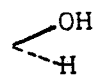

soit le groupement céto, les noyaux B, C et D portent éventuellement une ou plusieurs doubles liaisons et les noyaux A, B, C et D sont éventuellement substitués :

a) par une ou plusieurs fonctions hydroxyle ou cétone,

b) par un ou plusieurs atomes d'halogène,

c) par un ou plusieurs radicaux alkyles, linéaires ou ramifiés, renfermant de 1 à 10 atomes de carbone, éventuellement substitués par une fonction cétone et/ou par un radical acyloxy renfermant de 1 à 18 atomes de carbone et/ou par un radical alkyloxycarbonyl renfermant de 2 à 5 atomes de carbone,

d) par un ou plusieurs radicaux alkyloxy, linéaires ou ramifiés, renfermant de 1 à 4 atomes de carbone,

e) par un ou plusieurs radicaux acyloxy renfermant de 1 à 18 atomes de carbone,

f) par un ou plusieurs radicaux alkényles ou alkynyles renfermant de 2 à 4 atomes de carbone, le trait pointillé dans le cycle A représentant une double liaison éventuelle en 4(5), à l'action d'un agent de déshydrogénation tel que défini précédemment, pour obtenir le composé Δ1,4 correspondant de formule II

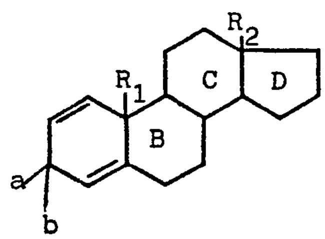

(II)

Lorsque $R_1$ représente un radical alkyle, il s'agit de préférence du radical méthyle ou éthyle.

Lorsque $R_1$ représente un radical alkyle substitué par une fonction oxygénée, il s'agit de préférence du radical hydroxy-méthyle ou hydroxy-éthyle, du radical formyle ou du radical acétyle.

Lorsque $R_1$ représente un radical alkyle substitué par une fonction azotée, il s'agit de préférence d'un groupement cyano ou du radical aminométhyle ou aminoéthyle.

Lorsque $R_1$ représente un radical alkyle substitué par un halogène, il s'agit de préférence d'un radical halométhyle $CH_2Hal$, dans lequel Hal représente un atome d'halogène, comme, par exemple, un atome de chlore, de fluor ou de brome.

Lorsque $R_1$ représente un radical alkényle, il s'agit de préférence du radical vinyle ou allyle.

Lorsque $R_1$ représente un radical alkynyle, il s'agit de préférence du radical éthynyle.

$R_2$ représente de préférence un radical méthyle ou éthyle.

Lorsque les noyaux B, C et D portent une ou plusieurs doubles liaisons, il s'agit de préférence de doubles liaisons en 9(11) et/ou 16(17) ou d'un système de doubles liaisons conjugées en 3(4) et 5(6) ou en 4(5) et 6(7).

Lorsque les cycles A, B, C et D sont substitués par une fonction hydroxyle, il s'agit de préférence d'une fonction hydroxyle en 3 ou 11.

Lorsque les cycles A, B, C et D sont substitués par une fonction cétone, il s'agit de préférence d'une fonction cétone en 3 ou en 11.

Lorsque les cycles A, B, C et D sont substitués par un atome d'halogène, il s'agit de préférence d'un atome de fluor, de chlore ou de brome, en position 6 ou 9α par exemple.

Lorsque les cycles A, B, C et D sont substitués par un ou plusieurs radicaux alkyle, il s'agit de préférence du radical méthyle ou éthyle en 2, 6, 7, en 16α ou en 16β, ou d'un radical alkyle en 17.

Lorsque les cycles A, B, C et D sont substitués par un radical alkyloxy, il s'agit de préférence d'un radical méthoxy ou éthoxy en 3 ou 11β.

Lorsque les cycles A, B, C et D sont substitués par un radical alkényle, il s'agit de préférence du radical vinyle ou allyle en position 11β par exemple.

Lorsque les cycles A, B, C et D sont substitués par un radical alkynyle, il s'agit de préférence du radical éthynyle en position 11β par exemple.

Par radical acyloxy, on entend de préférence un radical acétyloxy ou propionyloxy.

Par radical alkyloxy carbonyl, on entend de préférence un radical méthoxycarbonyl ou éthoxycarbonyl.

L'invention a notamment pour objet l'utilisation des agents de déshydrogénation de l'invention, caractérisée en ce que le produit de départ utilisé répond à la formule I pour laquelle

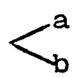

représente un groupement céto et $R_1$ et $R_2$ représentent un radical méthyle.

L'invention a tout particulièrement pour objet l'utilisation des agents de déshydrogénation de l'invention pour la déshydrogénation de composés de formule I dans laquelle le noyau D est substitué.

L'invention a naturellement tout particulièrement pour objet les utilisations des agents de déshydrogénation de l'invention, décrites dans la partie expérimentale exposée ci-après.

L'invention a également en particulier pour objet l'utilisation des agents de déshydrogénation de l'invention pour préparer les produits II figurant ci-après des produits I correspondants :

Produit I | Produit II

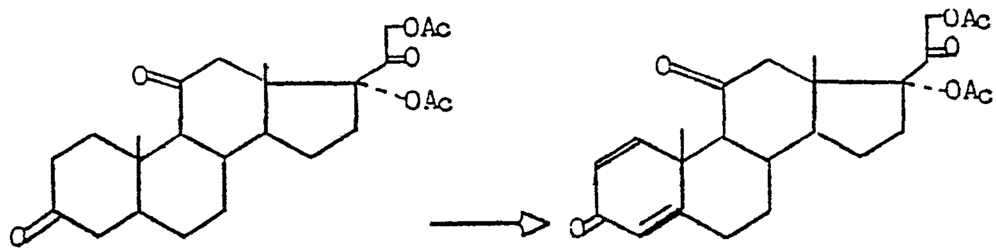

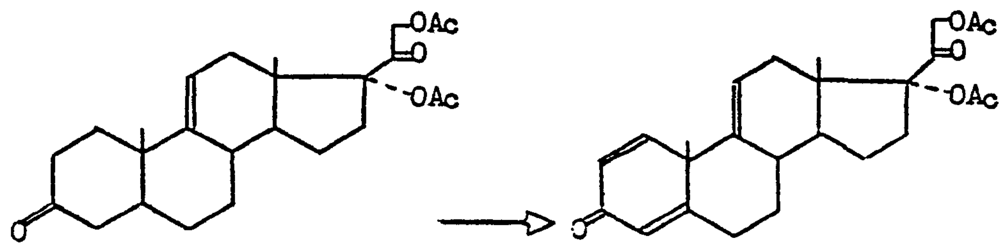

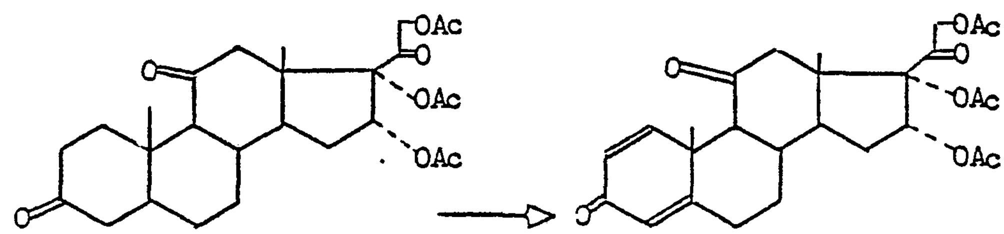

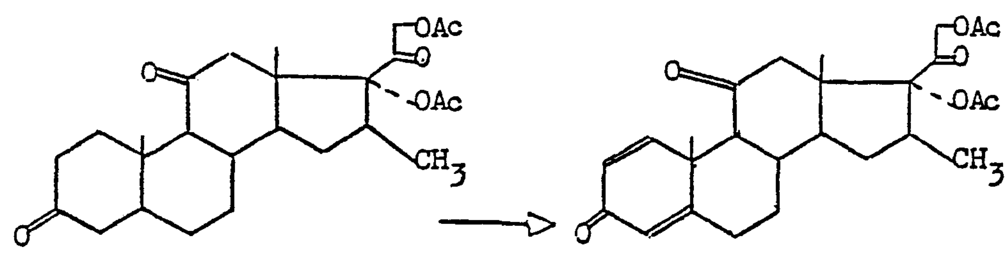

Produit I  Produit II

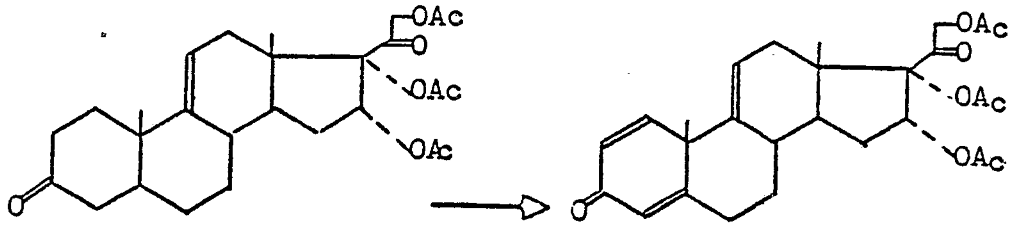

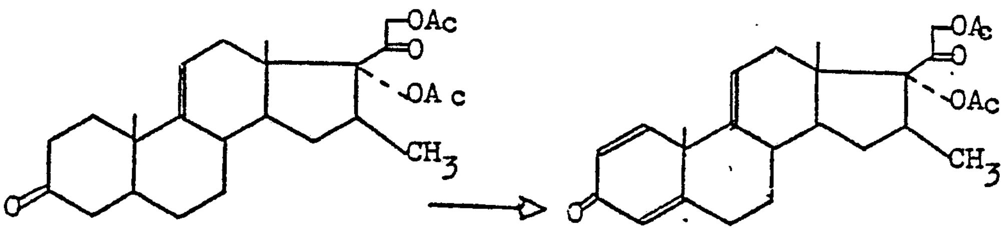

Les produits résultants de la déshydrogénation sont des produits chimiques connus en thérapeutique ou utilisés industriellement dans la synthèse de produits thérapeutiquement actifs : voir par exemple le brevet belge 545 877 ou le brevet belge 682 889.

Les exemples suivants illustrent l'invention sans toutefois la limiter :

## Exemple 1

17α acétoxy 16β méthyl — pregna 1,4,9(11) triène 3,20 dione.

On chauffe au reflux pendant 15 minutes, dans 20 $cm^3$ de toluène, 720 mg d'acide m-iodocybenzoïque et 52 mg de diphényl disélénure. On ajoute alors 300 mg de 17α-acéthoxy 16β méthyl 5βH pregna 9(11)ene 3,20 dione, et chauffe encore au reflux pendant 2 heures et demie.

On refroidit, extrait à l'aide d'une solution saturée de bicarbonate de sodium, lave à l'eau et sèche. On recristallise le produit obtenu dans un mélange d'acétone et d'héxane. On obtient ainsi 255 mg du produit recherché fondant à 230-233 °C. $\alpha_D^{19} = + 10°$ (c = 0,9 % $CHCl_3$).

On réduit les phases aqueuses par traitement à la température ambiante avec une solution saturée d'hydrosulfite de sodium pendant 1 heure. On extrait au toluène et récupère ainsi 47 mg (soit 90 %) de diphényldisélénure. On oxyde la phase aqueuse par un courant d'air pendant 1 heure pour détruire l'excès d'hydrosulfite et acidifie avec de l'acide sulfurique dilué. On obtient par filtration, lavage et séchage 550 mg d'acide méta-iodoxybenzoïque.

## Exemples 2 à 14

Le tableau suivant résume la préparation de produits II selon le procédé indiqué ci-dessus.

| | | Conditions de la Réaction | | | Produits II obtenus 1,4-dien-3-one | |
|---|---|---|---|---|---|---|
| Ex. | Produits I de départ | Temps (h) | Solvant/T °C | Catalyseur équivalent | Réactif en équivalent d'iodoxybenzène | Rendement |
| 2 | Cholestan-3-one | 24 | $S_1$ | $C_1$ (0,2) | $M_1$ (3,3) | 84 % |
| 3 | Cholestan-3β-ol | 24 | $S_1$ | $C_1$ (0,2) | $M_1$ (3,3) | 81 % |
| 4 | | 12 | $S_1$ | $C_1$ (0,2) + TsOH (trace) | $M_1$ (3,3) | 77 % |
| 5 | Androst-4-en-3,17-dione | 12 | $S_1$ | $C_1$ (0,16) | $M_1$ (3,6) | 72 % |
| 6 | Pregn-4-en-3,11,20 trione | 12 | $S_1$ | $C_1$ (0,18) | $M_1$ (3,3) | 51 % |
| 7 | Cholestan-3β-ol | 20 | $S_1$ | $C_1$ (0,2) | $M_2$ (3,3) | 74 % |
| 8 | | 3 | $S_2$ | $C_2$ (0,2) | $M_2$ (3,3) | 88 % |
| 9 | | 2 | $S_3$ | $C_2$ (0,2) | $M_2$ (3,3) | 79 % |
| 10 | | 16 | $S_2$ | $C_2$ (0,03) | $M_2$ (4,16) | 73 % |

(Suite)

| | | Conditions de la Réaction | | | | Produits II obtenus 1,4-dien-3-one |
|---|---|---|---|---|---|---|
| Ex. | Produits I de départ | Temps (h) | Solvant/T °C | Catalyseur équivalent | Réactif en équivalent d'iodoxybenzène | Rendement |
| 11 | 3α hydroxy-5β cholan oate de méthyle | 3 | $S_2$ | $C_2$ (0,2) | $M_2$ (3,3) | 84 % |
| 12 | 17α acétoxy-16β méthyl 5β pregna-9(11)-en 3,20 dione | 2,5 | $S_2$ | $C_2$ (0,2) | $M_2$ (3,2) | 86 % |
| 13 | 17α acétoxy-16α méthyl 5β pregn 9(11)-en 3,20 dione | 2,5 | $S_2$ | $C_1$ (0,14) | $M_2$ (3,0) | 75 % |
| 14 | cholestan-3-one | 3 | $S_3$ | $C_3$ (0,5) | $M_1$ (3,0) | 58 % |

Réactifs et solvants : $S_1$ benzène au reflux, $S_2$ toluène au reflux, $S_3$ chlorobenzène au reflux, $C_1$ anhydrique benzèneséléninique, $C_2$ diphényl disélénure, $C_3$ dioxyde de sélénium, $M_1$ iodoxybenzène, $M_2$ acide méta-iodoxybenzoïque.

**Revendications** (pour les Etats contractants : BE, CH, DE, FR, GB, IT, LI, LU, NL, SE)

1. A titre d'agents de déshydrogénation, le diphényl disélénure, et l'anhydride benzène séléninique lorsqu'ils sont utilisés en quantité catalytique, en présence d'un agent d'oxydation choisi dans le groupe constitué par l'iodoxy benzène, l'acide méta-iodoxybenzoïque et les composés de formule

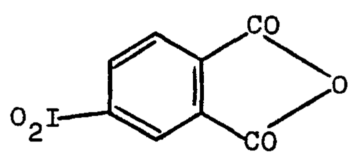

et

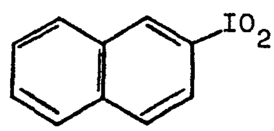

2. A titre d'agent de déshydrogénation selon la revendication 1, le diphényl disélénure utilisé en quantité catalytique en présence d'acide méta-iodoxybenzoïque.

3. Utilisation des agents de déshydrogénation selon l'une quelconque des revendications 1 et 2, caractérisée en ce que l'on soumet un composé de formule I

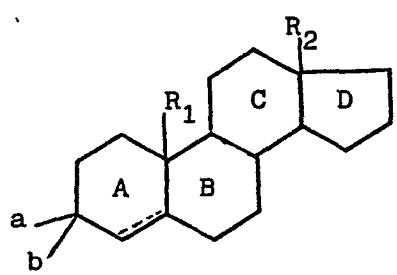

(I)

dans laquelle $R_1$ représente un atome d'hydrogène, un radical alkyle renfermant de 1 à 4 atomes de carbone éventuellement substitué par une fonction oxygénée ou azotée ou un atome d'halogène, ou $R_1$ représente un radical alkényle ou alkynyle renfermant de 2 à 4 atomes de carbone, $R_2$ représente un radical alkyle renfermant de 1 à 4 atomes de carbone, le radical

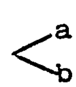

représente soit le groupement

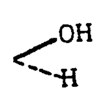

soit le groupement céto, les noyaux B, C et D portent éventuellement une ou plusieurs doubles liaisons et les noyaux A, B, C et D sont éventuellemant substitués :

a) par une ou plusieurs fonctions hydroxyle ou cétone,

b) par un ou plusieurs atomes d'halogène,

c) par un ou plusieurs radicaux alkyles, linéaires ramifiés, renfermant de 1 à 10 atomes de carbone, éventuellement substitués par une fonction cétone et/ou par un radical acyloxy renfermant de 1 à 18 atomes de carbone et/ou par un radical alkyloxycarbonyl renfermant de 2 à 5 atomes de carbone,

d) par un ou plusieurs radicaux alkyloxy, linéaires ou ramifiés renfermant de 1 à 4 atomes de carbone,

e) par un ou plusieurs radicaux acyloxy renfermant de 1 à 18 atomes de carbone,

f) par un ou plusieurs radicaux alkényle ou alkynyle renfermant de 2 à 4 atomes de carbone, le trait pointillé dans le cycle A représentant une double liaison éventuelle en 4(5), à l'action d'un agent de déshydrogénation tel que défini à l'une quelconque des revendications 1 ou 2, pour obtenir le composé Δ1,4 correspondant de formule II

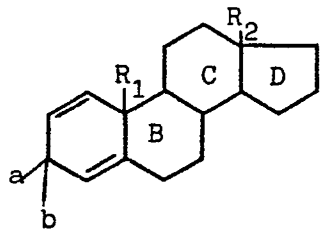

(II)

4. Utilisation selon la revendication 3, caractérisée en ce que le produit de départ utilisé répond à la formule I pour laquelle

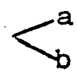

représente un groupement céto et $R_1$ et $R_2$ représentent un radical méthyle.

5. Utilisation selon la revendication 3 ou 4, caractérisée en ce que le noyau D est substitué par l'un des substituants définis à la revendication 3.

**Revendications** (pour l'Etat contractant AT)

1. Procédé de déshydrogénation caractérisé en ce qu'il consiste à utiliser le diphényl disélénure, ou l'anhydride benzène sélénique en quantité catalytique en présence d'un agent d'oxydation choisi dans le groupe constitué par l'iodoxy benzène, l'acide méta-iodoxybenzoïque et les composés de formule

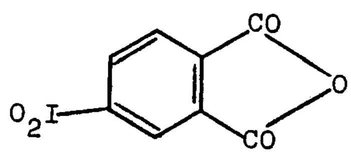

, et

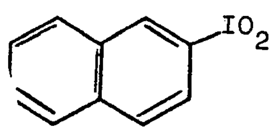

2. Procédé selon la revendication 1, caractérisé en ce que le diphényl disélénure est utilisé en quantité catalytique en présence d'acide méta-iodoxybenzoïque.

3. Procédé selon l'une quelconque des revendications 1 et 2 caractérisé en ce que le composé à déshydrogéner est un composé de formule I

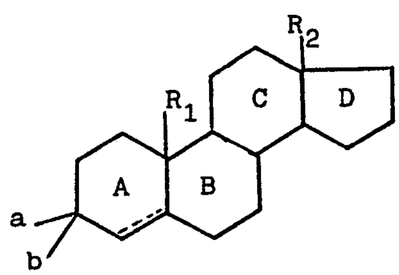

(I)

dans laquelle $R_1$ représente un atome d'hydrogène, un radical alkyle renfermant de 1 à 4 atomes de carbone éventuellement substitué par une fonction oxygénée ou azotée ou un atome d'halogène, ou $R_1$ représente un radical alkényle ou alkynyle renfermant de 2 à 4 atomes de carbone, $R_2$ représente un radical alkyle renfermant de 1 à 4 atomes de carbone, le radical

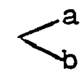

représente soit le groupement

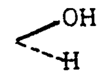

soit le groupement céto, les noyaux B, C et D portent éventuellement une ou plusieurs doubles liaisons et les noyaux A, B, C et D sont éventuellement substitués :

a) par une ou plusieurs fonctions hydroxyle ou cétone,

b) par un ou plusieurs atomes d'halogène,

c) par un ou plusieurs radicaux alkyles, linéaires ou ramifiés, renfermant de 1 à 10 atomes de carbone, éventuellement substitués par une fonction cétone et/ou par un radical acyloxy renfermant de 1 à 18 atomes de carbone et/ou par un radical alkyloxycarbonyl renfermant de 2 à 5 atomes de carbone,

d) par un ou plusieurs radicaux alkyloxy, linéaires ou ramifiés renfermant de 1 à 4 atomes de carbone,

e) par un ou plusieurs radicaux acyloxy renfermant de 1 à 18 atomes de carbone,

f) par un ou plusieurs radicaux alkényle ou alkynyle renfermant de 2 à 4 atomes de carbone, le trait pointillé dans le cycle A représentant une double liaison éventuelle en 4 (5), le composé obtenu étant le composé Δ1,4 correspondant de formule II

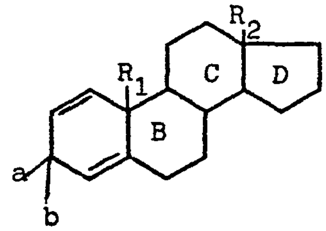

(II)

4. Procédé selon la revendication 3, caractérisé en ce que le produit de départ utilisé répond à la formule I pour laquelle

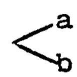

représente un groupement céto et $R_1$ et $R_2$ représentent un radical méthyle.

5. Procédé selon la revendication 3 ou 4, caractérisé en ce que le noyau D est substitué par l'un des substituants définis à la revendication 3.

6. Procédé selon l'une quelconque des revendications 3, 4 ou 5, caractérisé en ce que l'on utilise un agent de déshydrogénation tel que défini à l'une quelconque des revendications 1 et 2.

**Claims** (for the Contracting States : BE, CH, DE, FR, GB, IT, LI, LU, NL, SE)

1. As dehydrogenation agents, diphenyl diselenide and benzeneselenium anhydride, when they are used in catalytic quantity, in the presence of an oxidizing agent chosen from the group constituted by iodoxybenzene, meta-iodoxybenzoic acid and the compounds of formula

:

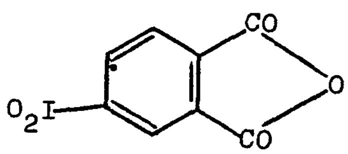

and

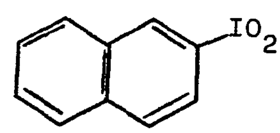

2. As a dehydrogenation agent according to claim 1, diphenyl diselenide used in catalytic quantity in the presence of meta-iodoxybenzoic acid.

3. Use of dehydrogenation agents according to either one of the claims 1 and 2, characterized in that there is submitted a compound with the formula I

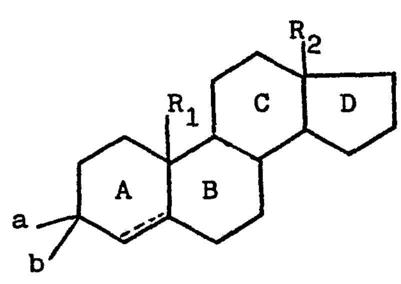

(I)

in which $R_1$ represents a hydrogen atom, an alkyl radical containing 1 to 4 carbon atoms, possibly substituted by an oxygenated or nitrated function, or a halogen atom, or $R_1$ represents an alkenyl or alkynyl radical containing 2 to 4 carbon atoms, $R_2$ represents an alkyl radical containing 1 to 4 carbon atoms, the radical

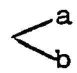

represents either the

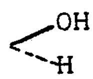

group or the keto group, the nuclei B, C and D possibly carry one or several double bonds and the nuclei A, B, C and D are possibly substituted ;

a) by one or more hydroxyl or ketone functions,

b) by one or more halogen atoms,

c) by one or more linear or branched alkyl radicals containing 1 to 10 carbon atoms, possibly substituted by a ketone function and/or by an acyloxy radical containing 1 to 18 carbon atoms, and/or by an alkyloxycarbonyl radical containing 2 to 5 carbon atoms,

d) by one or more linear or branched alkyloxy radicals containing 1 to 4 carbon atoms,

e) by one or more acyloxy radicals containing 1 to 18 carbon atoms,

f) by one or more alkenyl or alkynyl radicals containing 2 to 4 carbon atoms, the dotted line in ring A representing a possible double bond at 4(5), by the action of a dehydrogenation agent as defined in any one of the claims 1 or 2, to obtain the corresponding Δ1,4 compound with the formula II

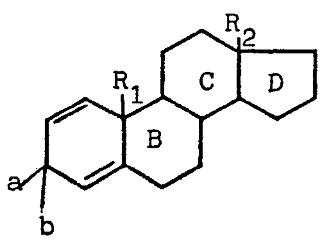

(II)

4. Use according to claim 3, characterized in that the starting product used answers to the formula I for which

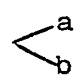

represents a keto group and $R_1$ and $R_2$ represent methyl radicals.

5. Use according to claim 3 or 4, characterized in that the nucleus D is substituted by one of the substituents defined in claim 3.

**Claims** (for the Contracting State AT)

1. Dehydrogenation process characterized in that it consists in using diphenyl diselenide, or benzeneselenium anhydride in catalytic quantity in the presence of an oxidizing agent chosen from the group constituted by iodoxybenzene, meta-iodoxybenzoic acid and the compounds with the formula

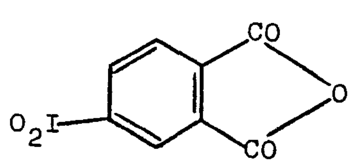

and

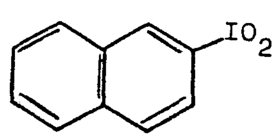

2. Process according to Claim 1, characterized in that the diphenyl diselenide is utilized in catalytic quantity in the presence of meta-iodoxybenzoic acid.

3. Process according to any one of the claims 1 and 2 characterized in that the compound to be dehydrogenated is a compound with the formula I

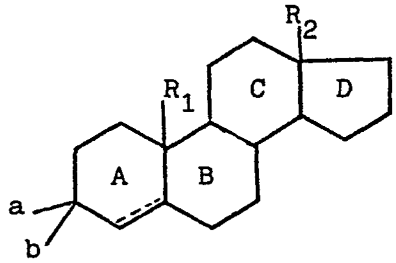

(I)

in which $R_1$ represents a hydrogen atom, an alkyl radical containing 1 to 4 carbon atoms, possibly substituted by an oxygenated or nitrated function, or a halogen atom, or $R_1$ represents an alkenyl or alkynyl radical containing 2 to 4 carbon atoms, $R_2$ represents an alkyl radical containing 1 to 4 carbon atoms, the radical

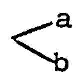

represents either the

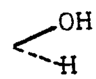

group or the keto group, the nuclei B, C and D possibly carry one or several double bonds and the nuclei A, B, C and D are possibly substituted ;

a) by one or more hydroxyl or ketone functions,

b) by one or more halogen atoms,

c) by one or more linear or branched alkyl radicals containing 1 to 10 carbon atoms, possibly substituted by a ketone function and/or by an acyloxy radical containing 1 to 18 carbon atoms and/or by an alkyloxycarbonyl radical containing 2 to 5 carbon atoms,

d) by one or more linear or branched alkyloxy radicals containing 1 to 4 carbon atoms,

e) by one or more acyloxy radicals containing 1 to 18 carbon atoms,

f) by one or more alkenyl or alkynyl radicals containing 2 to 4 carbon atoms, the dotted line in ring A representing a possible double bond at 4(5), the compound obtained being the corresponding Δ1,4 compound with the formula II

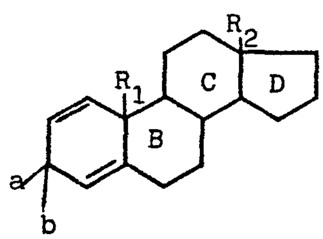

(II)

4. Process according to claim 3, characterized in that the starting product used answers to the formula I for which

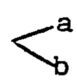

represents a keto group and $R_1$ and $R_2$ represent methyl radicals.

5. Process according to claim 3 or 4, characterized in that the nucleus D is substituted by one of the substituents defined in claim 3.

6. Process according to any one of the claims 3, 4 or 5, characterized in that a dehydrogenation agent is used as defined in any one of the claims 1 and 2.

**Patentansprüche** (für die Vertragsstaaten : BE, CH, DE, FR, GB, IT, LI, LU, NL, SE)

1. Als Dehydrierungsmittel Diphenyldiselenid und Benzclseleninsäureanhydrid, wenn sie in katalytischer Menge in Gegenwart eines Oxidationsmittels, ausgewählt unter Jodoxybenzol, m-Jodoxybenzoesäure und den Verbindungen der Formeln

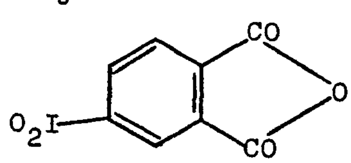

und

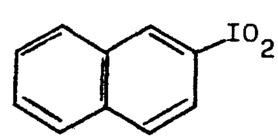

verwendet werden.

2. Als Dehydrierungsmittel gemäß Anspruch 1 das in katalytischer Menge in Gegenwart von m-Jodoxybenzoesäure verwendete Diphenyldiselenid.

3. Verwendung der Dehydrierungsmittel gemäß einem der Ansprüche 1 und 2, dadurch gekennzeichnet, daß man eine Verbindung der Formel I

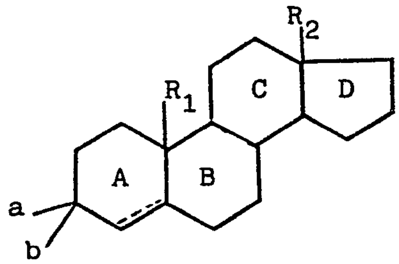

(I)

worin $R_1$ ein Wasserstoffatom, einen Alkylrest mit 1 bis 4 Kohlenstoffatomen, der gegebenenfalls durch eine Sauerstoff- oder Stickstoff-Funktion oder durch ein Halogenatom substituiert ist, bedeutet, oder $R_1$ einen Alkenyl- oder Alkinylrest mit 2 bis 4 Kohlenstoffatomen bedeutet, $R_2$ einen Alkylrest mit 1 bis 4 Kohlenstoffatomen darstellt, der Rest

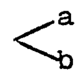

entweder die Gruppe

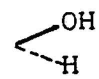

oder die Ketogruppe bedeutet, die Ringe B, C und D gegebenenfalls eine oder mehrere Doppelbindungen enthalten und die Ringe A, B, C und D gegebenenfalls substituiert sind durch :

a) eine oder mehrere Hydroxyl- oder Ketonfunktionen,

b) ein oder mehrere Halogenatome,

c) einen oder mehrere lineare oder verzweigte Alkylreste mit 1 bis 10 Kohlenstoffatomen, die gegebenenfalls durch eine Ketonfunktion und/oder durch einen Acyloxyrest mit 1 bis 18 Kohlenstoffatomen und/oder durch einen Alkoxycarbonylrest mit 2 bis 5 Kohlenstoffatomen substituiert sind,

d) einen oder mehrere lineare oder verzweigte Alkoxyreste mit 1 bis 4 Kohlenstoffatomen,

e) einen oder mehrere Acyloxyreste mit 1 bis 18 Kohlenstoffatomen,

f) einen oder mehrere Alkenyl- oder Alkinylreste mit 2 bis 4 Kohlenstoffatomen, wobei die gestrichelte Linie in dem Ring A eine etwaige Doppelbindung in 4(5)-Stellung bedeutet, der Einwirkung eines Dehydrierungsmittels, wie in einem der Ansprüche 1 oder 2 definiert, unterzieht, um die entsprechende Δ-1,4-Verbindung der Formel II

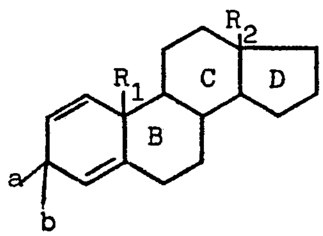

(II)

zu erhalten.

4. Verwendung gemäß Anspruch 3, dadurch gekennzeichnet, daß das verwendete Ausgangsprodukt der Formel I entspricht, worin

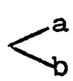

eine Ketogruppe bedeutet und $R_1$ und $R_2$ einen Methylrest darstellen.

5. Verwendung gemäß Anspruch 3 oder 4, dadurch gekennzeichnet, daß der Ring D durch einen der in Anspruch 3 definierten Substituenten substituiert ist.

**Patentansprüche** (für den Vertragsstaat AT)

1. Dehydrierungsverfahren, dadurch gekennzeichnet, daß es darin besteht, Diphenyldiselenid oder Benzolseleninsäureanhydrid in katalytischer Menge in Gegenwart eines Oxidationsmittels, ausgewählt unter Jodoxybenzol, m-Jodoxybenzoesäure und den Verbindungen der Formeln

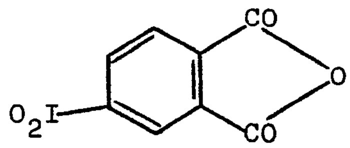

und

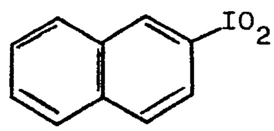

zu verwenden.

2. Verfahren gemäß Anspruch 1, dadurch gekennzeichnet, daß das Diphenyldiselenid in katalytischer Menge in Gegenwart von m-Jodoxybenzoesäure verwendet wird.

3. Verfahren gemäß einem der Ansprüche 1 und 2, dadurch gekennzeichnet, daß die zu dehydrierende Verbindung eine Verbindung der Formel I

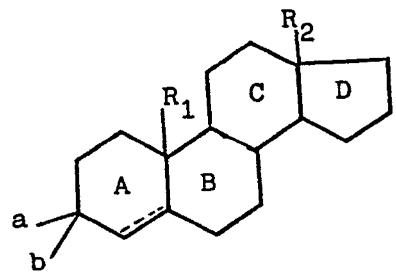

(I)

ist, worin $R_1$ ein Wasserstoffatom, einen Alkylrest mit 1 bis 4 Kohlenstoffatomen, der gegebenenfalls durch eine Sauerstoff- oder Stickstoff-Funktion oder durch ein Halogenatom substituiert ist, bedeutet oder $R_1$ einen Alkenyl- oder Alkinylrest mit 2 bis 4 Kohlenstoffatomen bedeutet, $R_2$ einen Alkylrest mit 1 bis 4 Kohlenstoffatomen bedeutet, der Rest

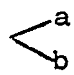

entweder die Gruppe

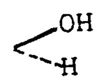

oder die Ketogruppe bedeutet, die Ringe B, C und D gegebenenfalls eine oder mehrere Doppelbindungen enthalten und die Ringe A, B, C und D gegebenenfalls substituiert sind durch

a) eine oder mehrere Hydroxyl- oder Ketonfunktionen,

b) ein oder mehrere Halogenatome,

c) einen oder mehrere lineare oder verzweigte Alkylreste mit 1 bis 10 Kohlenstoffatomen, die gegebenenfalls durch eine Ketonfunktion und/oder durch eine Acyloxygruppe mit 1 bis 18 Kohlenstoffatomen und/oder durch eine Alkoxycarbonylgruppe mit 2 bis 5 Kohlenstoffatomen substituiert sind,

d) einen oder mehrere lineare oder verzweigte Alkoxyreste mit 1 bis 4 Kohlenstoffatomen,

e) einen oder mehrere Acyloxyreste mit 1 bis 18 Kohlenstoffatomen,

f) einen oder mehrere Alkenyl- oder Alkinylreste mit 2 bis 4 Kohlenstoffatomen, wobei die gestrichelte Linie in dem Ring A eine etwaige Doppelbindung in 4(5)-Stellung bedeutet, wobei die erhaltene Verbindung die entsprechende Δ-1,4-Verbindung der Formel II

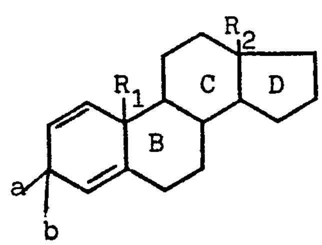

(II)

ist.

4. Verfahren gemäß Anspruch 3, dadurch gekennzeichnet, daß das verwendete Ausgangsprodukt der Formel I entspricht, worin

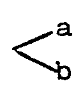

eine Ketogruppe bedeutet und $R_1$ und $R_2$ einen Methylrest darstellen.

5. Verfahren gemäß Anspruch 3 oder 4, dadurch gekennzeichnet, daß der Ring D durch einen der in Anspruch 3 definierten Substituenten substituiert ist.

6. Verfahren gemäß einem der Ansprüche 3, 4 oder 5, dadurch gekennzeichnet, daß man ein Dehydrierungsmittel, wie in einem der Ansprüche 1 und 2 definiert, verwendet.